# EUROPEAN PATENT APPLICATION

(11) **EP 4 280 813 A1**
(43) Date of publication of application: **22.11.2023**
(21) Application number: 23174374.1
(22) Date of filing: 19.05.2023
(51) Int. Cl.: H05B 1/02, H05B 6/64, F24C 7/00

(54) **SYSTEM AND METHOD FOR MOISTURE AND AMBIENT HUMIDITY LEVEL PREDICTION FOR FOOD DONENESS**

(30) Priority: 20.05.2022 US 202217749558
(71) Applicant: Whirlpool Corporation, Benton Harbor, MI 49022 (US)
(72) Inventor: Ehrenbeck, Blake W., 21024 Cassinetta di Biandronno (VA) (IT); Herndon, Seth, 21024 Cassinetta di Biandronno (VA) (IT); Lin, Chia-Hua, 21024 Cassinetta di Biandronno (VA) (IT); Wiatrak, Bruce M., 21024 Cassinetta di Biandronno (VA) (IT)
(74) Representative: PGA S.p.A.

(57) **Abstract**

A method for controlling a heating process may include recognizing a food class of a food item (602), defining a target doneness score for the food item based on at least one of the food class and a desired doneness level (604), receiving a food humidity level, receiving a cavity humidity level, receiving a cavity temperature (608), determining a current doneness score of the food item according to the food humidity level (610), the cavity humidity level and the cavity temperature, and utilizing the current doneness score and the target doneness score to control a heating system to cook the food item (118).

## Description

### FIELD OF DISCLOSURE

Disclosed herein are systems and methods for moisture and ambient humidity level prediction for food doneness.

### DESCRIPTION OF RELATED ART

Cooking appliances may include predefined cooking modes or cycles for specific food types, such as bake, roast, pizza, cookie, toast, popcorn, and bagel. These modes may be designed to allow a user to cook food items without setting multiple cooking parameters. For instance, a popcorn mode of a microwave oven may be defined to perform a predefined cycle of cooking at a specific wattage for a predefined period of time. Because food items vary in size, shape, and initial temperature, predefined cooking modes may not always provide optimal results.

### SUMMARY

A method for controlling a heating process may include recognizing a food class of a food item, defining a target doneness score for the food item based on at least one of the food class and a desired doneness level, receiving a food humidity level, receiving a cavity humidity level, receiving a cavity temperature, determining a current doneness score of the food item according to the food humidity level, the cavity humidity level and the cavity temperature, and utilizing the current doneness score and the target doneness score to control a heating system to cook the food item.

In one example, the method includes sending begin cooking instructions to the heating system to begin cooking the food item.

In one embodiment, the method includes sending end cooking instructions to the heating system responsive to the current doneness score reaching the target doneness score.

In another example, the method includes identifying the food class of the food item responsive to user input.

In one example, the method include retrieving the target doneness score from a threshold lookup table according to the food class.

In one embodiment, the food humidity level is determined based on a voltage difference between a sensed voltage of the food item and a fixed voltage.

In another example, the method includes retrieving the food humidity level from a lookup table according to the voltage difference.

In one example, the sensed voltage is provided by a pair of voltage probes (214) inserted into the food item.

A smart oven for controlling a heating process, may include at least one food humidity sensor for acquiring a food humidity level, at least one cavity humidity sensor for acquiring a cavity humidity level, at least one cavity temperature sensor for acquiring a cavity temperature, a heating system. The smart oven may also include a processor, programmed to receive the food humidity level, receive the cavity humidity level, receive the cavity temperature, determine a current doneness score of a food according to the food humidity level, the cavity humidity level and the cavity temperature, and utilize the current doneness score and a target doneness score to control the heating system to cook the food.

In one example, the processor is further programmed to: send begin cooking instructions to the heating system to begin cooking the food, and send end cooking instructions to the heating system responsive to the current doneness score reaching at least a minimum doneness likelihood threshold specified by the target doneness score.

In one embodiment, the processor is further programmed to identify a food class of the food responsive to user input.

In another example, the processor is further programmed to retrieve the target doneness score from a threshold lookup table according to the food class.

In one example, the food humidity sensor is a probe sensor having a pair of probes configured to be inserted into the food.

In another example, the probe sensor is configured to provide a sensed voltage and the processor is further programmed to determine the food humidity level based on a voltage difference between the sensed voltage of the food and a fixed voltage.

In one embodiment, the processor is further programmed to retrieve the food humidity level from a lookup table according to the voltage difference.

### BRIEF DESCRIPTION OF THE DRAWINGS

The embodiments of the present disclosure are pointed out with particularity in the appended claims. However, other features of the various embodiments will become more apparent and will be best understood by referring to the following detailed description in conjunction with the accompanying drawings in which:
FIG. 1 illustrates an example front perspective view of an oven configured to perform real-time automated cooking cycles;
FIG. 2 illustrates an example controller configured to operate the components of the smart oven to perform real-time automated cooking cycles;
FIG. 3 illustrates aspects of the architecture of a portion of the doneness application;
FIG. 4 illustrates an example block diagram and schematic of a moisture sensing system of the doneness application;
FIG. 5 illustrates an example chart; and
FIG. 6 illustrates an example process for controlling a heating process.

### DETAILED DESCRIPTION

As required, detailed embodiments of the present disclosure are disclosed herein; however, it is to be understood that the disclosed embodiments are merely exemplary of the disclosure that may be embodied in various and alternative forms. The figures are not necessarily to scale; some features may be exaggerated or minimized to show details of particular components. Therefore, specific structural and functional details disclosed herein are not to be interpreted as limiting, but merely as a representative basis for teaching one skilled in the art to variously employ the present disclosure.

Cooking meals is a part of daily life for many people. People generally prioritize taste, nutrition, cost, and ease of preparation when planning their meals. Yet, these attributes are often in conflict. Real-time automated cooking cycles make cooking meals easier, so users of cooking appliances can place more emphasis on taste and nutrition. Such cycles let users offload a significant part of the work, the actual cooking process, to the appliance. This allows the users to invest time in the more active steps of meal preparation, such as measuring and combining ingredients. Instead of the user monitoring the cooking appliance while the food cooks, a computer vision system may monitor the food on the user's behalf, allowing the user to reclaim that time for other activities.

A smart oven may offer assisted cycles for different food types. These cycles may be pre-programmed with certain modes, temperatures, and times designated for each food type. These modes may also be adjustable according to user inputs for quantity and preference (e.g., light/dark toast, rare/medium steak). Responsive to a user selecting a cycle and options, in some systems the oven's timer settings and other procedures may be predetermined before the cooking cycle begins. This inflexibility may reduce the effectiveness of the assisted cycles. In such systems, the user may be able to customize the cycle for the number of bread slices and the desired brownness, but cannot for example, specify the dimensions of the slices or the initial temperature, which affect how the food item may cook. Therefore, the assisted cycles may be unable to account for whether the bread was taken from the fridge or the room-temperature countertop, for instance.

Various cooking variables may affect the cooking process, such as the individual oven's hot spots, weather and humidity, size and material of the pan, moisture content of the ingredients, etc.

A machine-learning assisted smart cycle for an oven may predict a desired humidity level per food type as well as a rate of change. Once the desired humidity of the food is reached, the system may notify the user that cooking is complete. This may be applicable to baked goods, meats, casseroles, etc., where a certain humidity level is typically preferred to ensure doneness.

Various sensors, such as probes, may be used to observe the food as it cooks. Described herein is a doneness application configured to predict food doneness using a two-prong probe. The probe is configured to use a fixed voltage to measure a voltage difference of the food, which is then fed to an analog to digital converter. Humidity sensors within the oven cavity may also be used to detect an internal cavity moisture level, and measure this level over time. Based on the food type and the desired moisture level, the doneness level may be predicted and displayed to the user at the oven's interface, or at another user device, such as a mobile device. The desired moisture level may depend on the food type. Temperature sensors may also be used to detect a cavity temperature, or food temperature. The sensor data may be used to generate a score that is compared to a threshold score level specific to the food type.

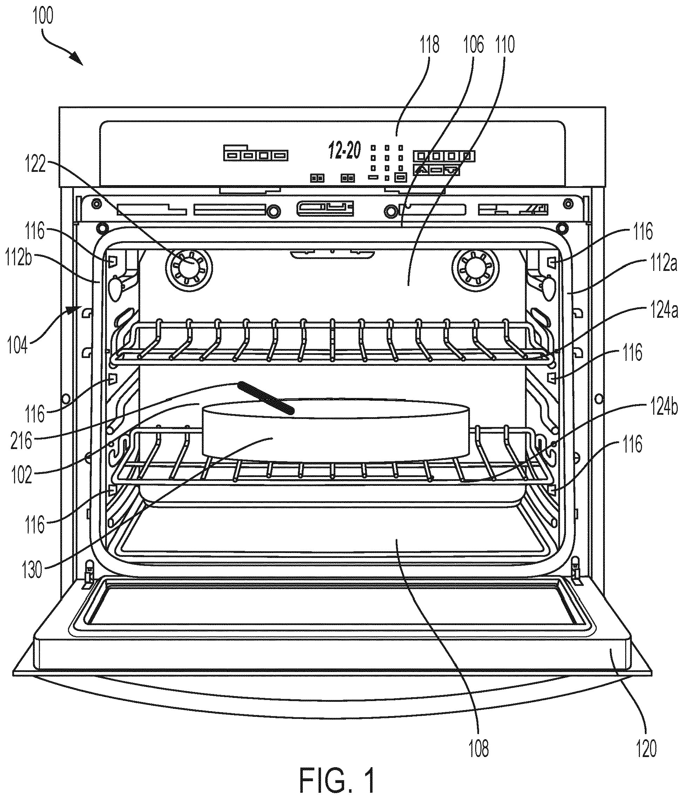

FIG. 1 illustrates an example front perspective view of a smart oven 100 configured to perform real-time automated cooking cycles. The smart oven 100 may be one of various cooking appliances, such as a conventional oven, a convection oven, a conduction oven, a microwave oven, a toaster oven. In some examples, the smart oven 100 may be a function-specific oven, such as a roaster oven, a pizza oven, etc. The smart oven 100 may be a standalone oven in some cases, while in other cases the oven may be built-in or a component of a combination oven and stove top.

The smart oven 100 may form a cabinet 104 and define a cavity 102 having a cavity top 106, cavity bottom 108, cavity back 110, and side walls 112. A door assembly 120 may be hinged at a front of the cavity bottom 108 to permit access to the cavity 102. The door assembly 120 may include a window and a handle and may hermetically seal the cavity when the door is in a closed position. It should be noted that this is an example, and smart ovens 100 with different types of doors may be used. For instance, a door may be hinged at a side instead of the bottom. A door sensor may be arranged on the door or the cavity 102 to detect an open and closed position of the door of the door assembly 120.

The cavity 102 may be configured to receive food items for cooking, baking, and/or broiling during a cooking cycle. The cavity 102 may also include temperature sensors 116 for determining the air temperature within the cavity 102 during cooking. The smart oven 100 may further include a user interface 118 configured to receive user input with respect to cycles or other oven operation. The user interface 118 may also provide information to the user such as cook time, temperature, etc.

The smart oven 100 may include a heating system 122 for heating the cavity 102 during cooking. The heating system 122 may include one or more heating elements, such as a gas heating element or an electric heating element. In one example, the heating system 122 may include a first heating element at the bottom of the cavity 102, and a second heating element at the top of the cavity 102. In yet a further example, heating elements may be arranged between the cabinet 104 and the cavity back 110 and/or the cavity top 106.

The smart oven 100 may include one or more racks 124 within the cavity 102 for supporting the food items during cooking. As shown by way of example in FIG. 1, the oven may include a top rack 124a and a bottom rack 124b (collectively referred to herein as racks 124). It should be noted that while two racks 124 are shown, smart ovens 100 with more or fewer racks 124 are possible. Regardless of quantity, the racks 124 may rest on side rails arranged along the side walls 112. The side rails may extend parallel or generally parallel with the cavity top 106 and cavity bottom 108 along the side walls 112 at spaced intervals. The side rails may extend up the height of the side walls 112 to allow for varying positions of the racks 124 within the cavity 102. For each side rail arranged on the first side wall 112a, a corresponding side rail is arranged on the opposite second side wall 112b (generally at the same relative height) so that the rack 124 may be evenly maintained on each side thereof.

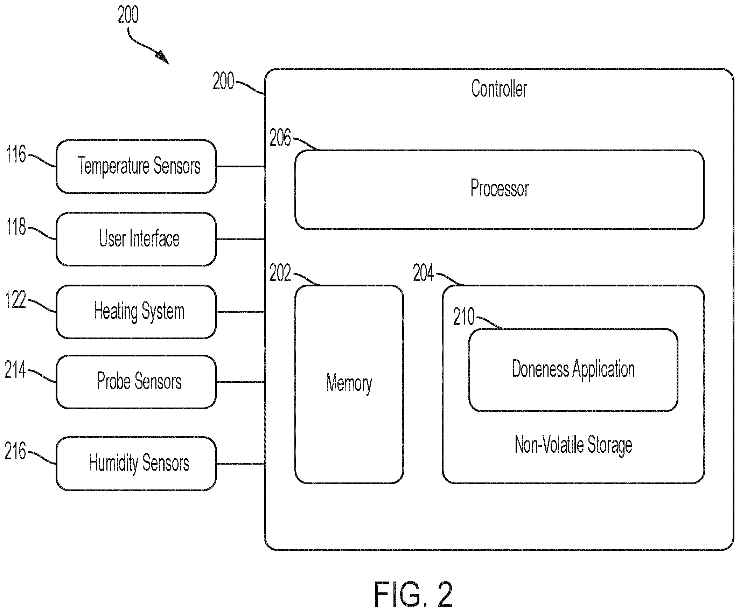

As explained, food may be placed in a container 130 and a sensor 216 may be arranged in the food to detect certain properties of the food as the food cooks. The sensor 216 may be a probe sensor 216 and is described in more detail with respect to FIG. 2.

FIG. 2 illustrates an example controller 200 configured to operate the components of the smart oven 100 to perform real-time automated cooking cycles. The controller 200 may include a memory 202, a non-volatile storage 204, and a processor 206. The non-volatile storage 204 may store operations for a doneness application 210.

The memory 202 may include a single memory device or a number of memory devices including, but not limited to, random access memory (RAM), volatile memory, non-volatile memory, static random access memory (SRAM), dynamic random access memory (DRAM), flash memory, cache memory, or any other device capable of storing information. The non-volatile storage 204 may include one or more persistent data storage devices such as a hard drive, optical drive, tape drive, non-volatile solid-state device, cloud storage or any other device capable of persistently storing information.

The processor 206 may include one or more microprocessors, micro-controllers, digital signal processors, microcomputers, central processing units (CPU), graphical processing units (GPU), tensor processing units (TPU), field programmable gate arrays, programmable logic devices, state machines, logic circuits, analog circuits, digital circuits, or any other devices that manipulate signals (analog or digital) based on computer-executable instructions residing in memory 202.

The processor 206 may be configured to read into memory 202 and execute computer-executable instructions residing in the non-volatile storage 204, such as those of the doneness application 210. Upon execution by the processor 206, the computer-executable instructions may cause the smart oven 100 to implement one or more of the algorithms and/or methodologies disclosed herein.

The controller 200 may be electrically connected to signaling interfaces of other components of the smart oven 100, thereby allowing the processor 206 of the controller 200 to manipulate the functions of the smart oven 100. For example, the controller 200 may be configured to receive user input from the user interface 118, such as requests to initiate a cooking cycle. The controller 200 may also be configured to control operation of the heating system 122, including to apply power to heating elements of the heating system 122 to warm the cavity 102, as well as to discontinue applying power to the heating element of the heating system 122. The controller 200 may also control a door lock to selectively control the latch mechanism of the door assembly 120 to prevent the door assembly 120 from being opened during the cycle.

The controller 200 may also be configured to receive input from other sensors to support the operation of the doneness application 210. This may include input from sensors such as the temperature sensors 116. This may also include input from one or more additional sensors such as probe sensors 214 and cavity humidity sensors 216.

The probe sensors 214 may be two-prong voltage sensors configured to measure the voltage difference of the food with respect to a fixed voltage. The prongs may be leads inserted into the food to be cooked. The voltage level may correspond to the humidity level and may be used to predict the humidity level of the food. The probe sensors 214 may be wired or wireless and may communicate with the doneness application 210 within the controller. The probe sensors 214 may also be referred to herein as food humidity sensors configured to acquire the food humidity level.

The fixed voltage may be set according to a predefined voltage level setting maintained within the memory 202.

The cavity humidity sensors 216 may be configured to provide data that may be used to determine the change of moisture content within the food as the cooking cycle progresses. In an example, the measure of humidity may be an amount of moisture contained within the food. In another example, the measure of humidity may be an amount of moisture identified within the cooking cavity during the cooking process. The cavity humidity sensors 216 may provide further context into the size and shape changes happening throughout the cooking process by relating them to a change in moisture. This change in moisture may allow for a more precise decision on the exact level of doneness through the cooking process.

Other sensors may also be included, such as temperature sensors that detect the internal temperature of food. Weight sensors may also be included and use load cells or pressure sensors integrated into the racks 124 to provide information about the weight of items in the smart oven 100. A change in this weight over time may indicated doneness. For example, a shrinking burger loses water weight as it cooks, and when that rate of loss slows, more browning is expected. The change between initial and final/current weight may also inform the cooking process, as heavier items may use more time to cook completely then smaller items.

In an example, the doneness application 210 may use the cavity humidity level, food humidity level, and cavity temperature and implement a recurrent model as executed by the processor 206 of the controller 200 of the smart oven 100 to determine food doneness.

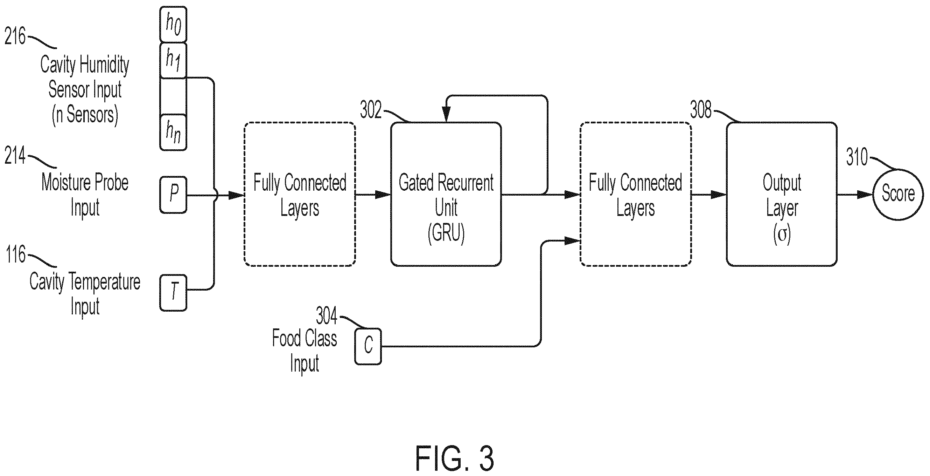

The doneness application 210 may receive these inputs of the food class and desired doneness level, and may utilize a lookup table maintained in the memory 202 of the controller 200 of the smart oven 100 to retrieve appliance control unit (ACU) begin cooking instructions and a target doneness score corresponding to at least one of food class and desired doneness level. In an example, the target doneness score may be a value scaled from 0 to 1. The begin cooking instructions may include temperature, convection, and/or other settings to control the heating system 122 of the smart oven 100. Further aspects of the architecture of a gated recurrent model (GRU) 302 are discussed below with respect to FIG. 3. The GRU may be part of a neural network.

FIG. 3 illustrates aspects of the architecture of a portion of the doneness application 210. The doneness application 210 may receive sensor data from the various sensors including the humidity sensors 216, probe sensors 214, and temperature sensors 116. Other inputs may also be received such as images, weight, food temperature, etc. These inputs may be used to produce the current doneness score 310. Further, the doneness application 210 may benefit from machine learning or artificial intelligence where the doneness application 210 is continually being 'trained' and refined.

The sensor data may be received by the GRU 302. The GRU 302 may receive the sensor data following transformations from fully connected layers. The GRU 302 may be used to perform analysis on the received data to establish certain correlations between humidity, voltage, temperature, etc.

In the examples herein, the GRU 302 may be a convolutional gated recurrent network, but other implementations are possible. As some other examples, the GRU 302 may be a non-convolutional GRU, a non-convolutional long-short term memory (LSTM) unit, a convolutional LSTM, or another type of recurrent unit that models temporal relationships.

An output layer 308 may receive a food class 304. In an example, the food class 304 may be indicated by confirming a suggested cycle presented by a recognition algorithm. The food class 304 may also be indicated by user selection of a specific food class or type. The user may also specify a desired doneness level, *e.g.,* through the user interface of the smart oven 100. In other examples, the desired doneness level may be retrieved from user preferences.

During the cooking cycle, sensor data continuously feed the GRU 302 with information about the state of the food being cooked. As explained, the GRU 302 may be part of a larger neural network. The doneness application 210 outputs a doneness score 310 with the same scale as a target doneness score (*e.g.,* also from 0 to 1). The doneness score 310 is compared with the target doneness score. If the current doneness score 310 is less than the target doneness score, the cooking cycle continues. If the scores are equal or the current doneness score 310 exceeds the target doneness score, the user is notified through the user interface or some other method (e.g., mobile notification). At this point, the smart oven 100 may send end cooking instructions to the heating system 122 to automatically turn off or switch to a "Keep Warm" mode, depending on the user's settings. The user can opt to continue cooking, start a new batch, or acknowledge that the cycle is complete.

If the user accepts the food when the current doneness score 310 matches the target doneness score, no change is made to the target doneness score. However, if the user directs the appliance to continue cooking the food, the GRU 302 may continue to generate a current doneness score 310 until the user completes the cycle. When complete, the smart oven 100 may revise the target doneness score. If this occurs, settings and lookup tables within the memory 202 may be updated for that in its lookup table for that food type and doneness setting. Adjacent target doneness scores the different levels of the same food type. Similarly, if the user ends the cooking cycle prior to the current doneness score 310 reaching its target doneness score, the smart oven 100 may revise the target score in memory 202. These adjustments may be made automatically, or only when confirmed by the user through a prompt (*e.g.* "Is this closer to your preference for "medium-well" steak?)

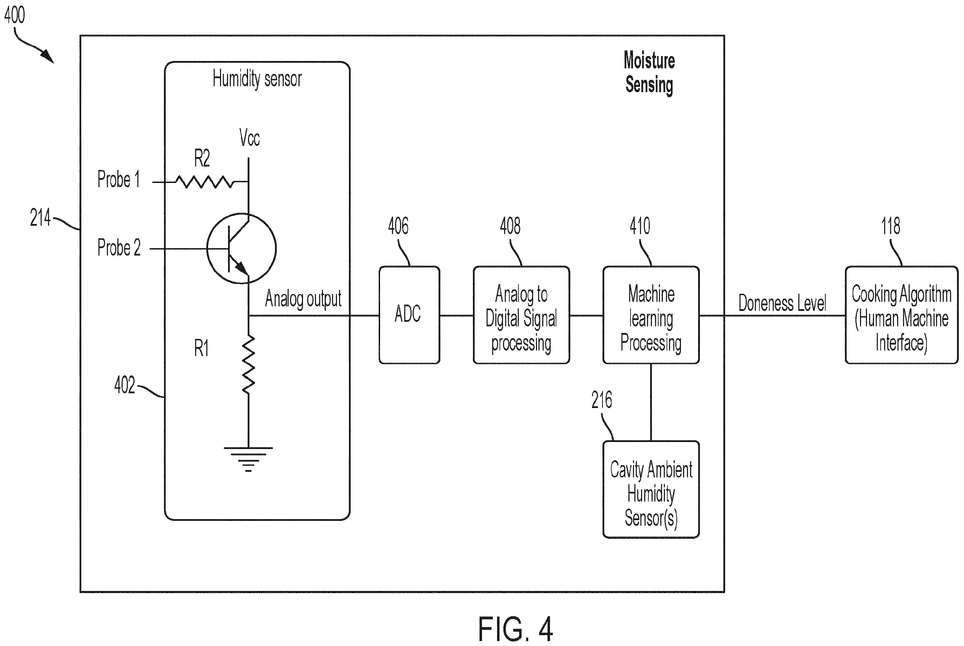

FIG. 4 illustrates an example block diagram and schematic of a moisture sensing system 400 of the doneness application 210. The moisture sensing system 400 may include a humidity sensor 402, which may include the probe sensors 214. The probe sensor 214, as explained, may include two probes or leads configured to provide an analog output, which is received at an analog to digital converter 406. Once converted, the digital signal is received at a processor 408 configured to process the signal. This may include comparing the digital voltage to a fixed or predefined voltage. The voltage difference may be determined and used to look up a relative humidity level. This may be done using certain look up tables maintained in the memory 202.

In one example, as the voltage detected by the probe sensor 214 increases, the humidity may be determined to decrease. A certain voltage level may inversely correlate to and therefore predict a certain humidity. The humidity may be the actual humanity in grams of moisture per cubic meter (g/m3). The predicted humidity may be used by the GRU 302 of FIG. 3 to determine the output score 310. The signal may then undergo machine learning processing 410 where certain training and predictions are made based on the relative humidity. The machine learning processing 410 may also receive cavity ambident humidity from the humidity sensors 216.

Such processing may produce a doneness level, or a score, which is used by the doneness application to determine whether the food is done as compared to the predefined doneness or score. Should the doneness level match or exceed the predefined doneness level, an output to the interface may be sent.

For example, when cooking baked goods such as cake or cookies in a baked goods food class 304, it may be most helpful to have the probe sensor 214 along with other sensors to determine the final doneness level 310 of the food. This may be because the final moisture content within the food at the end of cooking can determine whether the final state of the food is acceptably cooked or not. In each of the states of a dried-out cookie or moist cookie, the food is indeed cooked fully. However, the less moist cookie may be interpreted by the user as being over done while the moist cookie may be interpreted as being perfectly cooked.

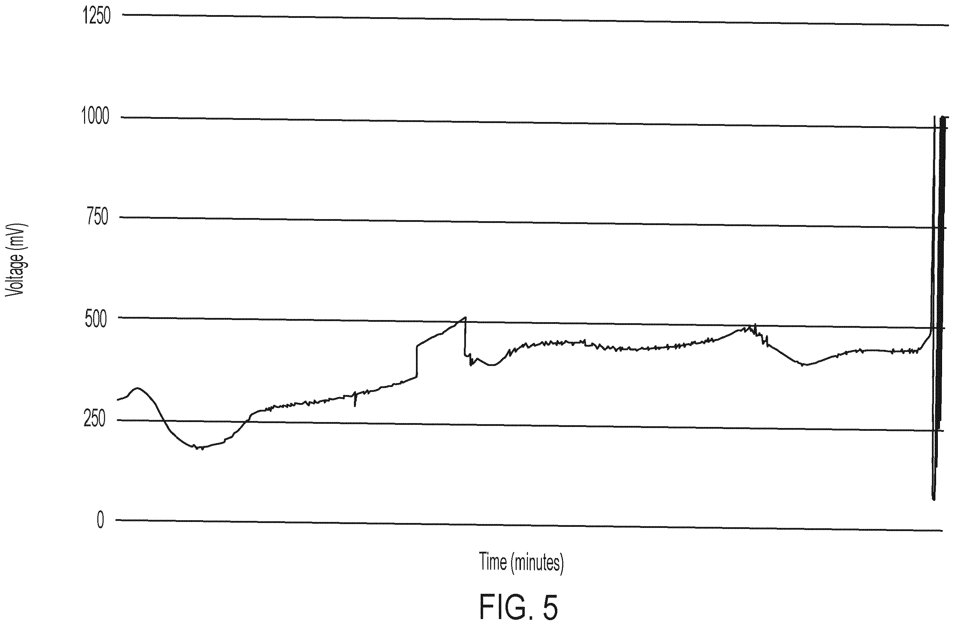

FIG. 5 illustrates an example chart of the voltage response over time. The voltage (mV) may correspond to the humidity and may vary over time (minutes). As the voltage increase, the humidity may decrease, thus indicating a level of doneness. As humidity decreases, food doneness level generally increases.

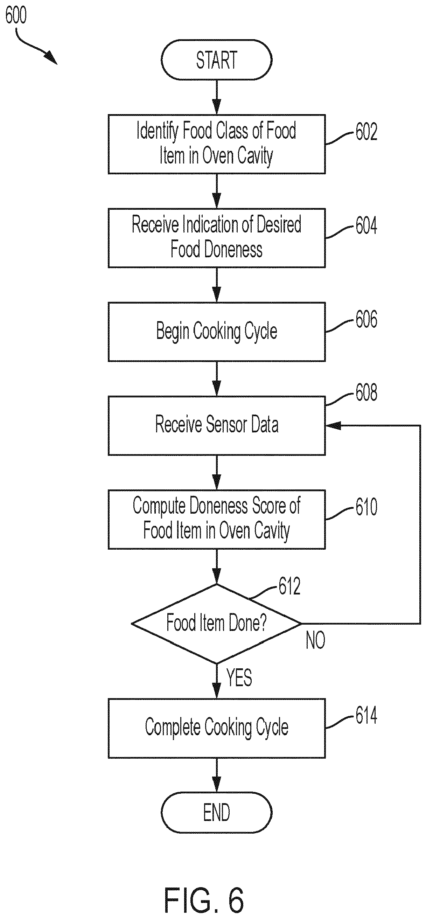

FIG. 6 illustrates an example process 600 for the use of the doneness application 210 to control the operation of the smart oven 100. In an example, the process 600 may be performed by the doneness application 210 implementing the GRU 302 as executed by the processor 206 of the controller 200 of the smart oven 100. The process 600 may be initiated, for example, by a user placing a food item into the cavity 102 of the smart oven 100 and closing the door of the smart oven 100.

At block 602, the smart oven 100 may identify the food class of the food item. In an example, the food class 304 may be indicated by user selection of a specific food class or type.

At block 604, the smart oven 100 receives an indication of a desired doneness level for the food item. In an example, the smart oven 100 may receive, from a user, a level of doneness from the user interface 118 of the smart oven 100. In some instances, the smart oven 100 may maintain user preferences with respect to the desired level of doneness and may use those preferences as the desired doneness level.

At block 606, the smart oven 100 begins a cooking cycle for the food item. For instance, the doneness application 210 may utilize the food class 304 and desired doneness level as inputs to a lookup table to retrieve begin cooking instructions to use to control the heating system 122. The doneness application 210 may similarly retrieve the target doneness score from the lookup table.

At block 608, the smart oven 100 receives sensor data. This sensor data may include, for example, cavity humidity level, food moisture level, and cavity temperature, as discussed with respect to FIG. 4. In an example, the smart oven 100 receives the sensor data from at least the humidity sensors 216, probe sensors 214 and temperature sensors 116.

At block 610, the smart oven 100 computes a doneness score 310 for the food item. Aspects of the computation of the doneness score 310 computation are described above with respect to FIG. 3. For example, each of the cavity humidity level, food moisture level, and cavity temperature may be used to determine the doneness level or score. At block 612, the smart oven 100 determines whether the food item is done. In an example, the smart oven 100 compares the doneness score 310 determined at block 610 with the desired doneness level. If the doneness score 310 indicates that the food item has not yet reached the desired doneness level, control returns to block 608. If the doneness 310 indicates that the food item is at least at the desired doneness level 306, control passes to operation 616 to complete the cooking cycle.

At block 614, the smart oven 100 completes the cooking cycle. In an example, the smart oven 100 may send the end cooking instructions to the heating system 122. This may, for example, discontinue operation of the heating system 122. Or, this may set the heating system 122 to a warming mode to keep the food item ready for use. In another example, the smart oven 100 may display or sound an alert to indicate that preparation of the food item is complete. The process 600 then ends.

While exemplary embodiments are described above, it is not intended that these embodiments describe all possible forms of the disclosure. Rather, the words used in the specification are words of description rather than limitation, and it is understood that various changes may be made without departing from the spirit and scope of the disclosure. Additionally, the features of various implementing embodiments may be combined to form further embodiments of the disclosure.

The flowcharts and block diagrams in the figures illustrate the architecture, functionality, and operation of possible implementations of systems, methods and computer program products according to various embodiments of the present disclosure. In this regard, each block in the flowchart or block diagrams may represent a module, segment, or portion of code, which comprises one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts, or combinations of special purpose hardware and computer instructions.

## Claims

1. A method (600) for controlling a heating process, comprising:
recognizing a food class of a food item (602);
defining a target doneness score for the food item based on at least one of the food class and a desired doneness level (604);
receiving a food humidity level (608);
receiving a cavity humidity level (608);
receiving a cavity temperature (608);
determining a current doneness score (310) of the food item according to the food humidity level, the cavity humidity level and the cavity temperature (610); and
utilizing the current doneness score (310) and the target doneness score to control a heating system (122) to cook the food item (118).

2. The method (600) of claim 1, further comprising:
sending begin cooking instructions to the heating system (122) to begin cooking the food item (606); and
sending end cooking instructions to the heating system (122) responsive to the current doneness score (310) reaching the target doneness score (614).

3. The method (600) of claim 1, further comprising identifying the food class of the food item responsive to user input (602).

4. The method (600) of claim 1, further comprising retrieving the target doneness score from a threshold lookup table (202) according to the food class.

5. The method (600) of claim 1, wherein the food humidity level is determined based on a voltage difference between a sensed voltage of the food item and a fixed voltage.

6. The method (600) of claim 5, further comprising retrieving the food humidity level from a lookup table according to the voltage difference.

7. The method (600) of claim 5, wherein the sensed voltage is provided by a pair of voltage probes (214) inserted into the food item.

8. A processor (206) programmed to perform the method of any one of claims 1-7.

9. A smart oven (100) for controlling a heating process, comprising:
at least one food humidity sensor (214) for acquiring a food humidity level;
at least one cavity humidity sensor (216) for acquiring a cavity humidity level;
at least one cavity temperature sensor (116) for acquiring a cavity temperature;
a heating system (122); and
a processor (206), programmed to
receive the food humidity level (608),
receive the cavity humidity level (608),
receive the cavity temperature (608),
determine a current doneness score (310) of a food according to the food humidity level, the cavity humidity level and the cavity temperature (610), and
utilize the current doneness score (310) and a target doneness score to control the heating system (122) to cook the food (118).

10. The smart oven (100) of claim 9, wherein the processor is further programmed to:
send begin cooking instructions to the heating system (122) to begin cooking the food (606); and
send end cooking instructions to the heating system (122) responsive to the current doneness score (310) reaching at least a minimum doneness likelihood threshold specified by the target doneness score (614).

11. The smart oven (100) of claim 9, wherein the processor (206) is further programmed to identify a food class of the food responsive to user input (602).

12. The smart oven (100) of claim 11, wherein the processor (206) is further programmed to retrieve the target doneness score from a threshold lookup table (202) according to the food class.

13. The smart oven (100) of claim 9, wherein the food humidity sensor (214) is a probe sensor (214) having a pair of probes configured to be inserted into the food.

14. The smart oven (100) of claim 13, wherein the probe sensor (214) is configured to provide a sensed voltage and the processor (206) is further programmed to determine the food humidity level based on a voltage difference between the sensed voltage of the food and a fixed voltage.

15. The smart oven (100) of claim 14, wherein the processor (206) is further programmed to retrieve the food humidity level from a lookup table according to the voltage difference.
